(19) Europäisches Patentamt
European Patent Office
Office européen des brevets

(11) Veröffentlichungsnummer : **0 214 932**
**B1**

(12) # EUROPÄISCHE PATENTSCHRIFT

(45) Veröffentlichungstag der Patentschrift : **28.09.88**

(51) Int. Cl.⁴ : **C 10 M135/20**, C 07 C149/36, C 07 C149/273, C 08 K 5/37

(21) Anmeldenummer : **86810370.6**

(22) Anmeldetag : **20.08.86**

(54) **Schwefelhaltige Phenolderivate enthaltende Schmiermittel- und Hydraulikflüssigkeitszusammensetzungen und neue schwefelhaltige Phenolderivate.**

(30) Priorität : **26.08.85 CH 3659/85**

(43) Veröffentlichungstag der Anmeldung : **18.03.87 Patentblatt 87/12**

(45) Bekanntmachung des Hinweises auf die Patenterteilung : **28.09.88 Patentblatt 88/39**

(84) Benannte Vertragsstaaten : **BE DE FR GB IT**

(56) Entgegenhaltungen :
**EP-A- 0 166 696**
**DE-A- 3 414 297**
**FR-A- 2 367 059**
**US-A- 4 108 831**

(73) Patentinhaber : **CIBA-GEIGY AG**
**Klybeckstrasse 141**
**CH-4002 Basel (CH)**

(72) Erfinder : **Wirth, Hermann O., Dr.**
**Lessingstrasse 24**
**D-6140 Bensheim 3 (DE)**

EP 0 214 932 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft schwefelhaltide Phenolderivate enthaltende Schmiermittel- und Hydraulikflüssigkeitszusammensetzungen, neue schwefelhaltige Phenolderivate, sowie die Verwendung von solchen Derivaten als Additive für Schmiermittel, Hydraulikflüssigkeiten und Elastomere.

Mineralischen und synthetischen Schmiermitteln werden üblicherweise Zusatzstoffe zur Verbesserung der Gebrauchseigenschaften zugesetzt. Von besonderer Bedeutung sind Additive, welche die zu schmierenden Teile vor Reibungsabnutzung schützen. An solche Additive wird die Anforderung gestellt, dass sie das Lasttragevermögen des Schmiermittels erhöhen, nicht korrodierend auf die zu schmierenden Metallteile wirken und eine gute Hitzebeständigkeit besitzen.

Hierfür werden heute vorzugsweise phosphor- und schwefelhaltige Verbindungen verwendet wie z. B. Dialkyldithiophosphate gemäss der DE-A 29 21 620.

Schwefelhaltige Phenolderivate und deren Herstellung sind z. B. aus der US-A 4 108 831 bekannt, wo ihre antioxidative Wirkung im Zusammenhang mit der Stabilisierung von Polymeren beschrieben wird.

Es wurde gefunden, dass bestimmte schwefelhaltige Phenolderivate Schmiermitteln und Hydraulikflüssigkeiten gute Hochdruck- und Verschleissschutz-Eigenschaften vermitteln.

Die vorliegende Erfindung betrifft Zusammensetzungen enthaltend ein Schmiermittel oder eine Hydraulikflüssigkeit und mindestens eine Verbindung der Formel I

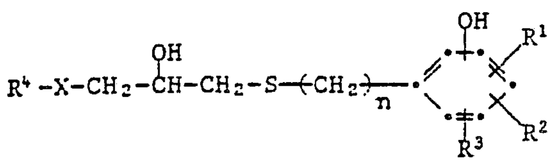

(I)

worin X für —S—, —O—, —CO—O—, —$CH_2$— oder —$NR^5$— steht und $R^5$ Wasserstoff oder $C_1$-$C_8$-Alkyl ist, n die Zahl 0, 1, 2 oder 3 bedeutet, $R^1$ und $R^2$ unabhängig voneinander Wasserstoff oder $C_1$-$C_{12}$-Alkyl sind, $R^3$ für Wasserstoff oder Methyl steht und $R^4$ $C_1$-$C_{22}$-Alkyl, $C_5$-$C_8$-Cycloalkyl, unsubstituiertes oder mit einem oder zwei $C_1$-$C_8$-Alkylresten substituiertes Aryl, $C_7$-$C_{12}$-Aralkyl oder die Gruppe

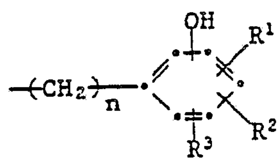

worin $R^1$, $R^2$, $R^3$ und n die angegebene Bedeutung haben, ist.

Hierbei hat X vorzugsweise die Bedeutung —S—, —O—, —$CH_2$— oder —CO—O—, insbesondere —S— oder —O—, und steht besonders bevorzugt für —S—. Handelt es sich bei X um die Gruppe —$NR^5$—, so ist $R^5$ bevorzugt $C_1$-$C_8$-Alkyl und besonders bevorzugt $C_1$-$C_4$-Alkyl. Bedeutet X —CO—O—, so ist das Carbonyl-C-Atom mit dem Rest $R^4$ verbunden.

n ist eine ganze Zahl 0, 1, 2 oder 3, bevorzugt 0 oder 1 und besonders bevorzugt 1.

$R^1$ und $R^2$ stellen vorzugsweise geradkettige oder verzweigte $C_1$-$C_{12}$-Alkylsubstituenten dar, die beispielsweise die Bedeutung Methyl, Ethyl, n-Propyl, Isopropyl, n-, sec.- oder tert.-Butyl, Pentyl, 1-Methylpentyl, n-Hexyl, 2-Ethylhexyl, n-Heptyl, 1-Methylheptyl, n-Octyl, geradkettiges oder verzweigtes Nonyl, Decyl, Undecyl oder Dodecyl haben.

$R^1$ und $R^2$ stehen unabhängig voneinander bevorzugt für Methyl oder einen geradkettigen oder verzweigten, vorzugsweise tertiären $C_4$-$C_8$-Alkylrest, wobei insbesondere mindestens einer der beiden Reste für den genannten tertiären Alkylrest steht.

In einer besonders bevorzugten Ausführungsform der Schmiermittel haben $R^1$ und $R^2$ in Verbindungen der Formel I unabhängig voneinander die Bedeutung Methyl oder tert.-Butyl, wobei insbesondere mindestens einer der beiden Reste für tert.-Butyl steht.

Die Reste $R^1$ und $R^2$ können verschieden oder gleich, bevorzugt gleich, sein.

$R^3$ hat vorzugsweise die Bedeutung Wasserstoff.

Besonders bevorzugt sind Schmiermittel, enthaltend eine Verbindung der Formel I, worin $R^1$ und $R^2$ unabhängig voneinander Methyl oder tert.-Butyl sind und $R^3$ Wasserstoff bedeutet.

$R^4$ in der Bedeutung $C_1$-$C_{22}$-Alkyl stellt einen geradkettigen oder verzweigten $C_1$-$C_{22}$-Alkylsubstituenten dar, der beispielsweise die Bedeutung Methyl, Ethyl, n-Propyl, Isopropyl, n-, sec.-, tert.-Butyl, Pentyl, 1-Methylpentyl, n-Hexyl, 2-Ethylhexyl, n-Heptyl, 1-Methylheptyl, n-Octyl, geradkettiges oder verzweigtes Nonyl, Decyl, Undecyl, Dodecyl, Tridecyl, Tetradecyl, Pentadecyl, Hexadecyl, Octadecyl, Eicosyl oder Docosyl hat.

$R^4$ steht vorzugsweise für einen geradkettigen oder verzweigten $C_2$-$C_{12}$-Alkylrest und besonders

bevorzugt für einen tertiären $C_4$-$C_9$-Alkylrest, wobei z. B. unter tert.-Octyl solch ein Rest verstanden werden soll, wie er für tertiäres Octyl-mercaptan in « Ullmanns Enzyklopädie der technischen Chemie, 4. Auflage, Band 23, Seite 181-182, Verlag Chemie, Weinheim » beschrieben ist, und somit X an ein tertiäres Kohlenstoffatom gebunden ist.

Bedeutet $R^4$ $C_5$-$C_8$-Cycloalkyl, handelt es sich z. B. um Cyclopentyl, Cyclohexyl, 4-Methylcyclohexyl oder Cyclooctyl ; $R^4$ ist hierbei vorzugsweise $C_5$-$C_6$-Cycloalkyl.

Ist $R^4$ einfach oder zweifach mit $C_1$-$C_8$-Alkylresten substituiertes Aryl, handelt es sich vorzugsweise um einfach oder zweifach mit geradkettigen oder verzweigten, $C_1$-$C_8$-Alkylresten, z. B. mit Methyl-, Ethyl-, n-Propyl-, Isopropyl-, n-, sec. oder tert.-Butyl, n-Pentyl, 1-Methylpentyl, n-Hexyl-, 2-Ethylhexyl-, n-Heptyl, 1-Methylheptyl- oder n-, sec. oder tert.-Octyl-Resten substituiertes Phenyl. $R^4$ ist hierbei bevorzugt ein Phenylrest mit einem oder zwei $C_1$-$C_4$-Alkyl-substituenten, wobei im Fall von zwei $C_1$-$C_4$-Alkyl Substituenten diese gleich oder verschieden, vorzugsweise gleich, sein können.

Bedeutet $R^4$ $C_7$-$C_{12}$-Aralkyl, so kann es sich z. B. um einen Benzyl-, α-Methylbenzyl-, α,α-Dimethylbenzyl-, 1-Phenylethyl-, 2-Phenylethyl-, 2-Phenylpropyl-, 3-Phenylpropyl-, 4-Phenylbutyl- oder Phenylhexyl-Rest handeln. Bevorzugt ist $R^4$ hierbei ein $C_7$-$C_{10}$-Aralkylsubstituent und besonders bevorzugt ein Benzylrest.

$R^4$ kann ausserdem die Gruppe

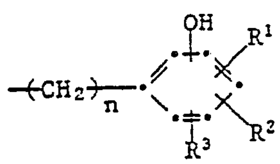

darstellen,
worin $R^1$, $R^2$, $R^3$ und n die angegebene Bedeutung haben.

Bevorzugt bedeutet $R_4$ $C_1$-$C_{22}$-, insbesondere $C_2$-$C_{18}$, vor allem $C_2$-$C_{12}$-Alkyl oder $C_5$-$C_8$-, insbesondere $C_5$-$C_6$-, vor allem $C_6$-Cycloalkyl ; vor allem aber $C_4$-$C_{12}$-Alkyl.

In den Verbindungen der Formel I kann sich die phenolische Hydroxygruppe in 2-, 3- oder 4-Stellung zum Rest $R^4$—X—$CH_2$—CH(OH)—$CH_2$—S—$(CH_2)_n$— befinden, wobei die 2- und 4-Stellung und insbesondere die 4-Stellung bevorzugt ist.

Die Reste $R^1$ und $R^2$ sind vorzugsweise in ortho-Stellung zur phenolischen Hydroxygruppe angeordnet.

Bevorzugt werden Zusammensetzungen, enthaltend ein Schmiermittel und wenigstens eine Verbindung der Formel I, wobei in den Verbindungen der Formel I X —S—, —O—, —$CH_2$— oder —CO—O—, insbesondere —S— oder —O—, vor allem —S—, bedeutet, n 0 oder 1 ist, $R^1$ und $R^2$ unabhängig voneinander für Wasserstoff, Methyl oder tertiäres $C_4$-$C_8$-Alkyl stehen, $R^3$ Wasserstoff ist und $R^4$ $C_2$-$C_{12}$-Alkyl, $C_5$-$C_6$-Cycloalkyl, unsubstituiertes oder mit einem oder zwei $C_1$-$C_4$-Alkylresten substituiertes Aryl oder $C_7$-$C_{10}$-Aralkyl darstellt.

Ferner sind jene Zusammensetzungen zu erwähnen, die ein Schmiermittel und mindestens eine Verbindung der Formel I enthalten, worin X für —S—, —O—, —$CH_2$— oder —CO—O— steht, n 0 oder 1 ist, die phenolische OH-Gruppe in 4-Stellung steht, $R_1$ und $R_2$ die ortho-Stellungen zur phenolischen OH-Gruppe besetzen und unabhängig voneinander $C_1$-$C_6$-Alkyl bedeuten, $R_3$ Wasserstoff ist und $R_4$ $C_2$-$C_{12}$-Alkyl oder Cyclohexyl bedeutet.

Die Verbindungen der Formel I sind teilweise neu. Ein weiterer Gegenstand der Erfindung sind daher neue Verbindungen der Formel I, worin X —S—, —O—, —CO—O— oder —$CH_2$— ist, n die Zahl 0, 1, 2 oder 3 bedeutet, $R^1$ und $R^2$ unabhängig voneinander Wasserstoff oder $C_1$-$C_{12}$-Alkyl sind, $R^3$ für Wasserstoff oder Methyl steht und $R^4$ $C_1$-$C_{22}$-Alkyl, $C_5$-$C_8$-Cycloalkyl, unsubstituiertes oder mit einem oder zwei $C_1$-$C_8$-Alkylresten substituiertes Aryl, $C_7$-$C_{12}$-Aralkyl oder, falls n 1, 2 oder 3 ist, auch die Gruppe

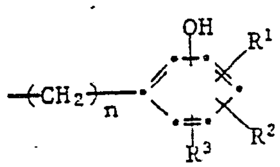

worin $R^1$, $R^2$ und $R^3$ die angegebene Bedeutung haben, ist. Hierbei gelten für $R^1$, $R^2$, $R^3$, $R^4$, X und n die zuvor geschilderten Bedeutungen und Bevorzugungen.

Bevorzugt sind Verbindungen der Formel I, worin X —S—, —O—, —$CH_2$— oder —CO—O— bedeutet, n 0 oder 1 ist, $R^1$ und $R^2$ unabhängig voneinander für Wasserstoff, Methyl oder tertiäres $C_4$-$C_8$-Alkyl stehen, $R^3$ Wasserstoff ist und $R^4$ $C_2$-$C_{12}$-Alkyl, $C_5$-$C_6$-Cycloalkyl, unsubstituiertes oder mit einem oder zwei $C_1$-$C_4$-Alkylresten substituiertes Aryl oder $C_7$-$C_{10}$-Aralkyl darstellt.

Auch bei den neuen Verbindungen der Formel I sind jene bevorzugt, worin $R^4$ $C_1$-$C_{22}$-Alkyl (insbesondere $C_2$-$C_{18}$-, vor allem $C_2$-$C_{12}$-Alkyl) oder $C_5$-$C_8$-Cycloalkyl (insbesondere $C_5$-$C_6$-, vor allem $C_6$-Cycloalkyl) bedeuten, wobei $C_4$-$C_{12}$-Alkyl besonders bevorzugt ist ; ferner jene, worin X —O— oder —S—, insbesondere letzteres bedeutet ; ferner solche, worin die phenolische OH-Gruppe in 4-Stellung steht, vor allem jene, worin $R_1$ und $R_2$ unabhängig voneinander Methyl oder tert.-Butyl bedeuten und die beiden ortho-Stellungen zur OH-Gruppe besetzen. Dabei trägt vorzugsweise mindestens eine dieser ortho-Stellungen einen tert.-Butylrest.

Besonders bevorzugt sind jene neuen Verbindungen der Formel I, worin X für —S—, —O—, —$CH_2$— oder —CO—O— steht, n 0 oder 1 ist, die phenolische OH-Gruppe in 4-Stellung steht, $R_1$ und $R_2$ die ortho-Stellungen zur phenolischen OH-Gruppe besetzen und unabhängig voneinander $C_1$-$C_6$-Alkyl bedeuten, $R_3$ Wasserstoff ist und $R_4$ $C_2$-$C_{12}$-Alkyl oder Cyclohexyl bedeutet.

Beispiele für Verbindungen der Formel I sind :

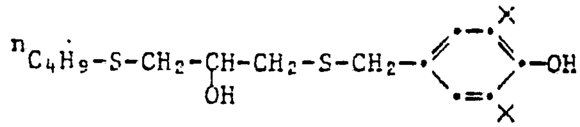

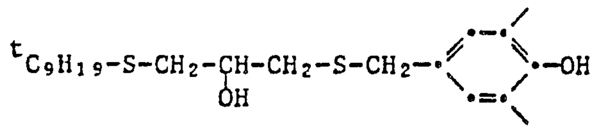

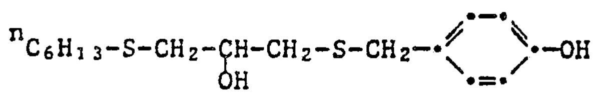

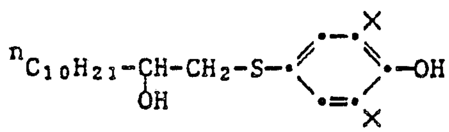

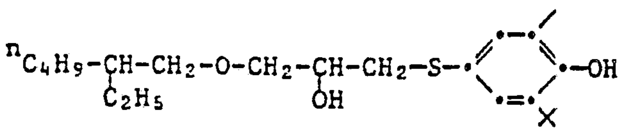

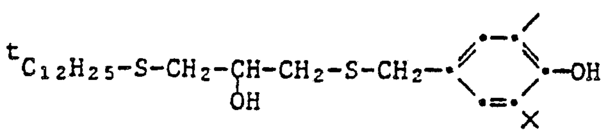

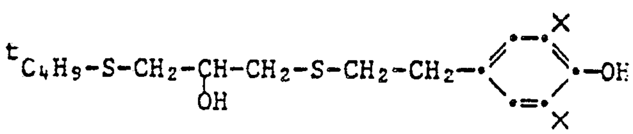

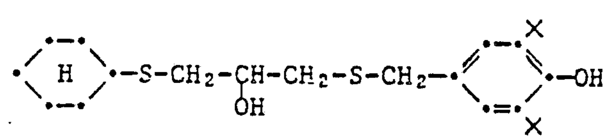

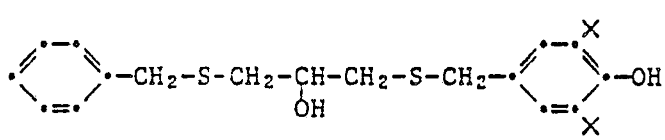

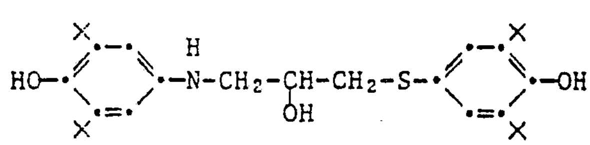

(Fortsetzung)

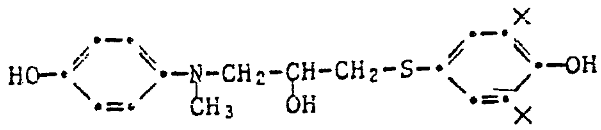

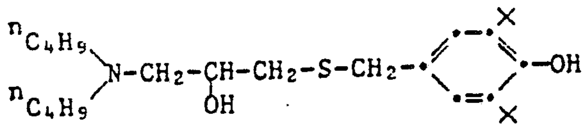

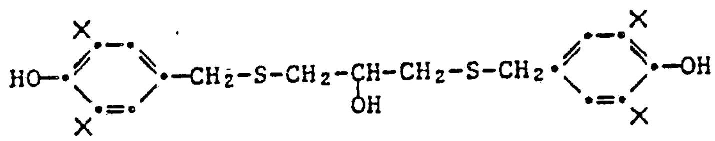

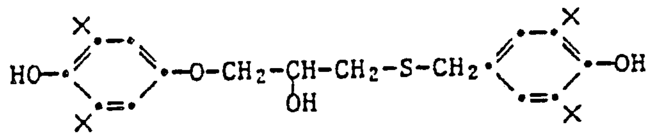

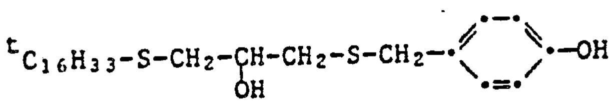

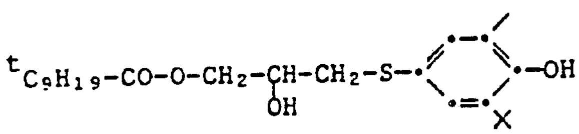

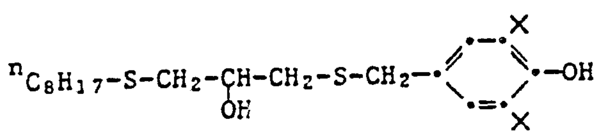

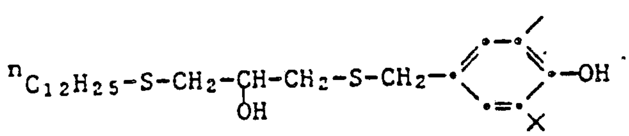

Die Verbindungen der Formel I können in an sich bekannter Weise, z. B. durch Umsetzung einer Glycidylverbindung der Formel II

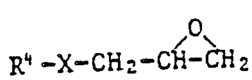

(II)

mit einer sulfidischen Verbindung der Formel III

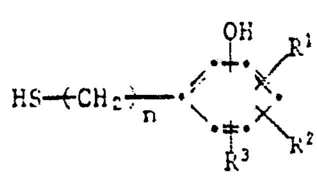

(III)

erhalten werden.

Die Verbindungen der Formel I können auch z. B. mittels Umsetzung einer Verbindung

$R^4$ — XH

mit einem phenolischen Glycidylthioetherderivat der Formel IV

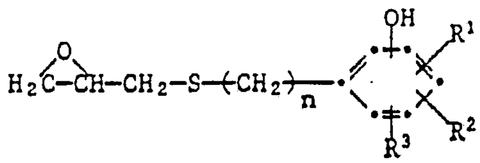

(VI)

hergestellt werden.

Hierbei haben $R^1$, $R^2$, $R^3$, $R^4$, X und n jeweils die zuvor angegebene Bedeutung.

Die Verbindungen der Formel II sind in an sich bekannter Weise, z. B. durch folgende Reaktion, zugänglich :

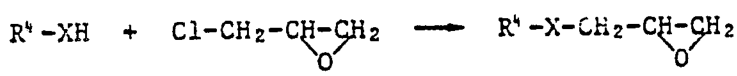

(II)

Die Verbindungen der Formel III sind z. B. aus den US-A 4 108 831 oder 4 165 333 bekannt, oder können z. B. entsprechend der EP-A 35472 oder analog G. Scott, Mater. Plast. Elastomeri 1977, S. 298-301 hergestellt werden.

Die Verbindungen der Formel IV sind z. B. aus T. Fujisawa et al., J. Polym. Sci. Polym. Lett. Ed. 12, S. 557-559 (1974) teilweise bekannt, oder können analog hergestellt werden.

Eine weitere Möglichkeit zur Herstellung der Verbindungen der Formel I, worin n = 1, besteht darin, ein Phenol der Formel

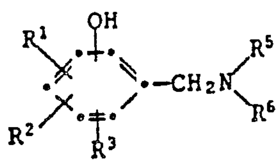

(V)

mit einer Verbindung der Formel

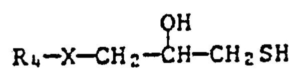

(VI)

umzusetzen. Auch diese Reaktion ist an sich bekannt und z. B. in der US-A 2 417 118 beschrieben. Die Ausgangsmaterialien der Formeln V und VI sind ebenfalls bekannt und können nach üblichen Methoden erhalten werden. Die Verbindungen der Formel V können z. B. durch Umsetzung eines Phenols der Formel

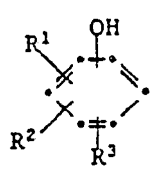

mit Formaldehyd oder einer Formaldehyd freisetzenden Verbindung und einem Amin der Formel $HNR^5R^6$ erhalten werden. Dabei bedeuten $R^5$ und $R^6$ wie in Formel V beispielsweise H. Alkyl (z. B. $C_1$-$C_6$-Alkyl), substituiertes Alkyl (z. B. mit OH, CH), Benzyl, Cycloalkyl, oder zusammen mit dem N-Atom einen 5- oder 6-gliedrigen gesättigten Heterocyclus. Bevorzugt bedeuten $R^5$ und $R^6$ $C_1$-$C_6$-Alkyl.

Mit den erfindungsgemässen schwefelhaltigen Phenolderivaten werden Schmiermittel- und Hydraulikflüssigkeit-Additive bereitgestellt, die sich durch gute Hochdruck-, Verschleissschutz- und Antioxidans-Eigenschaften auszeichnen. Die Verbindungen der Formel I wirken schon in sehr geringen Mengen in Schmiermitteln und Hydraulikflüssigkeiten. So zeigen z. B. mineralische und synthetische Schmieröle, sowie deren Gemische und Hydraulikflüssigkeiten, welche mit 0,01 bis 5 Gew.-%, bezogen auf das Schmiermittel, und vorzugsweise 0,05 bis 3 Gew.-% einer Verbindung der Formel I ausgestattet sind, ausgezeichnete Eigenschaften. Die in Frage kommenden Schmiermittel sind dem Fachmann geläufig, und z. B. in « Schmierstoffe und verwandte Produkte » (Verlag Chemie, Weinheim, 1982) beschrieben.

Besonders geeignet sind die Zusätze der Formel I für den Einsatz in nichtautomatischen, und

insbesondere in automatischen Getrieben von Automobilen. Ausserdem können sie hervorragende Dienste in Motorenölern, Diesellokomotivmotorenölen oder Turbinenölen leisten.

Die Erfindung betrifft daher auch die Verwendung von Verbindungen der Formel I sowie der neuen Verbindungen der Formel I als Additive für mineralische und synthetische Schmiermittel. Hierbei gelten für $R^1$, $R^2$, $R^3$, $R^4$, X und n die zuvor genannten Bevorzugungen.

Die Schmierstoffe und Hydraulikflüssigkeiten können zusätzlich andere Additive enthalten, die zugegeben werden, um die Grundeigenschaften von Schmierstoffen noch weiter zu verbessern ; dazu gehören : Antioxidantien, Metallpassivatoren, Rostinhibitoren, Viskositätsindex-Verbesserer, Stockpunkterniedriger, Dispergiermittel, Detergentien, Hochdruck-Zusätze und Antiverschleiss-Additive.

Beispiele für weitere phenolische Antioxidantien

1. Alkylierte Monophenole

2,6-Di-tert-butyl-4-methylphenol
2,6-Di-tert-butylphenol
2-tert-Butyl-4,6-dimethylphenol
2,6-Di-tert-butyl-4-ethylphenol
2,6-Di-tert-butyl-4-n-butylphenol
2,6-Di-tert-butyl-4-iso-butylphenol
2,6-Di-cyclopentyl-4-methylphenol
2-(α-Methylcyclohexyl-4,6-dimethylphenol
2,6-Di-octadecyl-4-methylphenol
2,4,6-Tri-cyclohexylphenol
2,6-Di-tert-butyl-4-methoxymethylphenyl
o-tert-Butylphenol

2. Alkylierte Hydrochinone

2,6-Di-tert-butyl-4-methoxyphenol
2,5-Di-tert-butyl-hydrochinon
2,5-Di-tert-amyl-hydrochinon
2,6-Diphenyl-4-octadecyloxyphenol

3. Hydroxylierte Thiodiphenylether

2,2′-Thio-bis-(6-tert-butyl-4-methylphenol)
2,2′-Thio-bis-(4-octylphenol)
4,4′-Thio-bis-(6-tert-butyl-3-methylphenol)
4,4′-Thio-bis-(6-tert-butyl-2-methylphenol)

4. Alkyliden-Bisphenole

2,2′-Methylen-bis-(6-tert-butyl-4-methylphenol)
2,2′-Methylen-bis-(6-tert-butyl-4-ethylphenol)
2,2′-Methylen-bis-[4-methyl-6-(α-methylcyclohexyl)-phenol]
2,2′-Methylen-bis-(4-methyl-6-cyclohexylphenol)
2,2′-Methylen-bis-(6-nonyl-4-methylphenol)
2,2′-Methylen-bis-(4,6-di-tert-butylphenol)
2,2′-Ethyliden-bis-(4,6-di-tert-butylphenol)
2,2′-Ethyliden-bis-(6-tert-butyl-4-iso-butylphenol)
2,2′-Methylen-bis-[6-(α-methylbenzyl)-4-nonylphenol]
2,2′-Methylen-bis-[6-(α,α-dimethylbenzyl)-4-nonylphenol]
4,4′-Methylen-bis-(2,6-di-tert-butylphenol)
4,4′-Methylen-bis-(6-tert-butyl-2-methylphenol)
1,1-Bis-(5-tert-butyl-4-hydroxy-2-methylphenyl)-butan
2,6-Di-(3-tert-butyl-5-methyl-2-hydroxybenzyl)-4-methylphenol
1,1,3-Tris-(5-tert-butyl-4-hydroxy-2-methylphenyl)-3-n-dodecyl-mercaptobutan
Ethylenglycol-bis-[3,3-bis-(3′-tert-butyl-4′-hydroxyphenyl)-butyrat]
Di-(3-tert-butyl-4-hydroxy-5-methylphenyl)-dicyclopentadien
Di-[2-(3′-tert-butyl-2′-hydroxy-5′-methyl-benzyl)-6-tert-butyl-4-methyl-phenyl-terephthalat.

5. Benzylverbindungen

1,3,5-Tri-(3,5-di-tert-butyl-4-hydroxybenzyl)-2,4,6-trimethylbenzol

Di-(3,5-di-tert-butyl-4-hydroxybenzyl)-sulfid
3,5-Di-tert-butyl-4-hydroxybenzyl-mercaptoessigsäure-isooctylester
Bis-(4-tert-butyl-3-hydroxy-2,6-dimethylbenzyl)-dithiol-terephthalat
1,3,5-Tris-(3,5-di-tert-butyl-4-hydroxybenzyl)-isocyanurat
1,3,5-Tris-(4-tert-butyl-3-hydroxy-2,6-dimethylbenzyl)-isocyanurat
3,5-Di-tert-butyl-4-hydroxybenzyl-phosphonsäure-dioctadecylester
3,5-Di-tert-butyl-4-hydroxybenzyl-phosphonsäure-monoethylester
Calcium-saltz.

6. Acylaminophenole

4-Hydroxy-laurinsäureanilid
4-Hydroxy-stearinsäureanilid
2,4-Bis-octylmercapto-6-(3,5-di-tert-butyl-4-hydroxyanilino)-s-triazin
N-(3,5-di-tert-butyl-4-hydroxyphenyl)-carbaminsäureoctylester:

7. Ester der β-(3,5-Di-tert-butyl-4-hydroxyphenyl)-propionsäure

mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

8. Ester der β-(5-tert-butyl-4-hydroxy-3-methylphenyl)-propionsäure

mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

9. Amide der β-(3,5-Di-tert-butyl-4-hydroxyphenyl)-propionsäure, wie z. B.

N,N'-Di-(3,5-di-tert-butyl-4-hydroxyphenylpropionyl)-hexamethylendiamin
N,N'-Di-(3,5-di-tert-butyl-4-hydroxyphenylpropionyl)-trimethylendiamin
N,N'-Di-(3,5-di-tert-butyl-4-hydroxyphenylpropionyl)-hydrazin.

Beispiele für aminische Antioxidantien :

N,N'-Di-isopropyl-p-phenylendiamin
N,N'-Di-sec-butyl-p-phenylendiamin
N,N'-Bis(1,4-dimethyl-pentyl)-p-phenylendiamin
N,N'-Bis(1-ethyl-3-methyl-pentyl)-p-phenylendiamin
N,N'-Bis(1-methyl-heptyl)-p-phenylendiamin
N,N'-Diphenyl-p-phenylendiamin
N,N'-Di-(naphtyl-2)-p-phenylendiamin
N-Isopropyl-N'-phenyl-p-phenylendiamin
N-(1,3-Dimethyl-butyl)-N'-phenyl-p-phenylendiamin
N-(1-Methyl-heptyl)-N'-phenyl-p-phenylendiamin
N-Cyclohexyl-N'-phenyl-p-phenylendiamin
4-(p-Toluol-sulfonamido)-diphenylamin
N,N'-Dimethyl-N,N'-di-sec-butyl-p-phenylendiamin
Diphenylamin
4-Isopropoxy-diphenylamin
N-Phenyl-1-naphthylamin
N-Phenyl-2-naphthylamin
octyliertes Diphenylamin
4-n-Butylaminophenol
4-Butyrylamino-phenol
4-Nonanoylamino-phenol
4-Dodecanoylamino-phenol
4-Octadecanoylamino-phenol

Di-(4-methoxy-phenyl)-amin
2,6-Di-tert-butyl-4-dimethylamino-methyl-phenol
2,4'-Diamino-diphenylmethan
4,4'-Diamino-diphenylmethan
N,N,N',N'-Tetramethyl-4,4'-diamino-diphenylmethan
1,2-Di-[(2-methyl-phenyl)-amino]-ethan
1,2-Di-(phenylamino)-propan
(o-Tolyl)-biguanid
Di-[4-(1',3'-dimethyl-butyl)-phenyl)amin
tert-octyliertes N-Phenyl-1-naphthylamino
Gemisch aus mono- und dialkylierten tert-Butyl-/tert-Octyldiphenylaminen.

Beispiele für Metallpassivatoren sind :

für Kupfer, Z. B. :
Benztriazol, Tetrahydrobenztriazol, 2-Mercaptobenzthiazol, 2,5-Di-mercaptothiadiazol, Salicylidenpropylendiamin, Salze von Salicylaminoguanidin.

Beispiele für Rost-Inhibitoren sind :

a) Organische Säuren, ihre Ester, Metallsalze und Anhydride, z. B. : N-Oleoyl-sarcosin, Sorbitanmono-oleat, Blei-naphthenat, Dodecenylbersnteinsäure-anhydrid, Alkenylbernsteinsäure-Halbester, 4-Nonylphenoxy-essigsäure.
b) Stickstoffhaltige Verbindungen, z. B. :
I. Primäre, sekundäre oder tertiäre aliphatische oder cycloaliphatische Amine und Amin-Salze von organischen und anorganischen Säuren, z. B. öllösliche Alkylammoniumcarboxylate.
II. Heterocyclische Verbindungen, z. B. :
Substituierte Imidazoline und Oxazoline.
c) Phosphorhaltige Verbindungen, z. B. :
Aminsalze von Phosphorsäurepartialestern.
d) Schwefelhaltige Verbindungen, z. B. :
Barium-dinonylnaphthalin-sulfonate, Calciumpetroleum-sulfonate.

Beispiele für Viskositätsindex-Verbesserer sind z. B.

Polymethacrylate, Vinylpyrrolidon/Methacrylat-Copolymere,
Polybutene, Olefin-Copolymere, Styrol/Acrylat-Copolymere.

Beispiele für Stockpunkterniedriger sind z. B. :

Polymethacrylat, alkylierte Naphthalinderivate.

Beispiele für Dispergiermittel/Tenside sind z. B. :

Polybutenylbernsteinsäure-imide, Polybutenylphosphonsäurederivate, basische Magnesium-, Calcium-, und Bariumsulfonate und -phenolate.

Beispiele für Verschleissschutz-Additive sind z. B. :

Schwefel und/oder Phosphor und/oder Halogen enthaltende Verbindungen, wie geschwefelte pflanzliche Oele, Zinkdialkyldithio-phosphat, Tritolyl-phosphat, chlorierte Paraffine, Alkyl- und Aryldisulfide.

Die neuen Verbindungen der Formel I eignen sich ausserdem sehr gut als Additive (Stabilisatoren) für Elastomere, welche sie gegen Zerstörung und Abbaureaktionen stabilisieren, die insbesondere durch oxidative Prozessde hervorgerunfen werden. Die vorliegende Erfindung betrifft daher, neben deren Verwendung als Additive für Schmiermittel, auch die Verwendung der neuen Verbindungen der Formel I als Additive (Stabilisatoren) für Elastomere. Ferner betrifft die vorliegende Erfindung neben Schmiermittelzusammensetzungen, die neue Verbindungen der Formel I enthalten, auch Zusammensetzungen, die ein Elastomer und mindestens eine neue Verbindung der Formel I enthalten.

Als Elastomere kommen z. B. folgende Materialien in Betracht :

A) Polydiene, wie beispielsweise Polybutadien, Polyisopren oder Polychloropren ; Blockpolymere, wie beispielsweise Styrol/Butadien/Styrol, Styrol/Isopren/Styrol oder Acrylnitril/Butadien-Copolymere.

B) Copolymere von Mono- oder Diolefinen untereinander oder mit anderen Vinylmonomeren, wie z. B. Aethylen-Alkylacrylat-Copolymere, Aethylen-Alkylmethacrylat-Copolymere, Aethylen-Vinylacetat-Copolymere sowie Terpolymere von Aethylen mit Propylen und einem Dien, wie Hexadien, Dicyclopentadien oder Aethylidennorbornen.

C) Halogenhaltige Polymere, wie z. B. Polychloropren, Chlorkautschuk, chloriertes oder chlorsulfoniertes Polyäthylen, Epichlorhydrin-Homo- und -Copolymere, Chlortrifluoroäthylen-Copolymere, Polymere aus halogenhltigen Vinylverbindungen, wie z. B. Polyvinylidenchlorid, Polyvinylidenfluorid ; sowie deren Copolymere, wie Vinylchlorid-Vinylidenchlorid, Vinylchlorid-Vinylacetat oder Vinylidenchlorid-Vinylacetat.

D) Polyurethane, die sich von Polyäthern, Polyestern und Polybutadien mit endständigen Hydroxylgruppen einerseits und aliphatischen oder aromatischen Polyisocyanaten andererseits ableiten sowie deren Vorprodukte.

E) Naturkautschuk.

F) Mischungen (Polyblends) der vorgenannten Polymeren.

G) Wässrige Emulsionen natürlicher oder synthetischer Kautschuke, wie z. B. Naturkautschuk-Latex oder Latices von carboxylierten Styrol-Butadien-Copolymeren.

Gegebenenfalls liegen diese Elastomere als Latices vor und können als solche stabilisiert werden.

Bevorzugt sind Zusammensetzungen, welche als Elastomeres ein Polydien, wie Polybutadien-Kautschuk, ein halogenhaltiges Polymer, wie Polyvinylidenfluorid, oder ein Polyurethan enthalten.

Die erfindungsgemässen Zusammensetzungen enthalten zweckmässig 0,01-10 Gew.-% mindestens einer neuen Verbindung der Formel I, bezogen auf das Elastomer, insbesondere 0,05-5,0 Gew.-%. Gemische von Stabilisatoren der Formel I können ebenfalls eingesetzt werden.

In der Praxis können die neuen Verbindungen Phenole der Formel I auch in Elastomeren zusammen mit anderen Stabilisatoren eingesetzt werden.

Als weitere Additive, mit denen zusammen die erfindungsgemäss verwendeten Stabilisatoren eingesetzt werden können, sind beispielsweise zu nennen :

1. Weitere Antioxidantien

Als Beispiele für Antioxidantien kommen jene in Betracht, die weiter oben unter den Punkten 1. bis 9. unter der Ueberschrift « Beispiele für weitere phenolische Antioxidantien » in Zusammenhang mit der Aufzählung von fakultativen Bestandteilen der erfindungsgemässen Schmiermittelzusammensetzungen angeführt sind.

2. UV-Absorber und Lichtschutzmittel

2.1. 2-(2'-Hydroxypehnyl)-benztriazole, wie z. B. das 5'-Methyl-, 3',5'-Di-tert.butyl-, 5'-tert.Butyl-, 5'-(1,1,3,3-Tetramethyl-butyl)-, 5-Chlor-3',5'-di-tert.butyl-, 5-Chlor-3'-tert;butyl-56'-methyl-, 3'-sec.Butyl-5'-tert.butyl, 4'-Octoxy-, 3',5'-Di-tert.-amyl-, 3',5'-Bis-(α,α-dimethylbenzyl)-Derivat.

2.2. 2-Hydroxybenzophenone, wie z. B. das 4-Hydroxy-, 4-Methoxy-, 4-Octoxy-, 4-Decyloxy-, 4-Dodecyloxy-, 4-Benzyloxy-, 4,2,4'-Tri-hydroxy-, 2'-Hydroxy-4,4'-dimethoxy-Derivat.

2.3. Ester von gegebenenfalls substituierten Benzoesäuren, wie z. B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-tert.butyl-4-hydroxybenzoesäure-2,4-di-tert.butylphenylester, 3,5-Di-tert.butyl-4-hydroxybenzoesäurehexadecylester.

2.4. Acrylate, wie z. B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z. B. Nickelkomplexe des 2,2'-Thio-bis-[4-(1,1,3,3-tetramethylbutyl)-phenols], wie der 1 : 1- oder der 1 : 2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern wie vom Methyl- oder Ethylester, Nickelkomplexe von Ketoximen, wie von 2-Hydroxy-4-methyl-phenyl-undecylketonoxim, Nickelkomplexe des 1-Phenyl-4-lauroyl-5-hydroxypyrazols, gegebenenfalls mit zusätzlichen Liganden.

2.6. Sterisch gehinderte Amine, wie z. B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bersteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-betantetracarbonsäure, 1,1'-(1,2-Ethandiyl)-bis-(3,3,5,5-tetramethyl-piperazinon).

2.7. Oxalsäurediamide, wie z. B. 4,4'-Di-octyloxy-oxanilid, 2,2'-Di-octyloxy-5,5'-di-tert.butyl-oixanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o- und p-Methoxy- sowie von o- und p-Ethoxy-di-substituierten Oxaliniden.

3. Metalldesaktivatoren, wie z. B. N,N'-Diphenyloxalsäurediamid, N-Salicylal-N'-salicyloylhydrazin, N,N'-Bis-salicyloylhydrazin, N,N'-Bis-(3,5-di-tert,butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B. Triphenylphosphit, Diphenylalkylphosphite, Phenyldialkylphosphite, Tri-(nonylpehnyl)-phosphit, Trilaurylphosphit, Trioctadecylphosphit, Distearyl-pentaerythritdiphosphit, Tris-(2,4-di-tert.butylphenyl)-phosphit, Diisodecylpentaeryrhtrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy-2,4,8,10-tetraoxa-3,9-diphosphaspiro[5.5]undecan.

5. Peroxidzerstörende Verbindungen, wie z. B. Ester der β-Thio-dipropionsäure, beispielsweise der Lauryl-, Stearyl-, Myristyl- oder Tridecylester, Mercapatobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z. B. Kupfersalze in Kombination mit Jodiden und/oder Phosphorverbindungen und Salze des zweiwertigen Mangans.

7. Basische Co-Stalilisatoren, wie z. B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z. B. 4-tert.Butylbenzoesäure, Adipinsäure, Diphenylessigsäure.

9. Füllstoffe und Verstärkungsmittel, wie z. B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z. B. Weichmacher, Gleitmittel, Emulgatoren, Pigmente, Optische Aufheller, Flammschutzmittel, Antistatika, Treibmittel.

Die Einarbeitung der neuen Verbindungen der Formel I kann beispielsweise durch Einmischen derselben und gegebenenfalls weiterer Additive nach den in der Technik übligen Methoden, vor oder während der Formgebung, oder auch durch Aufbringen der gelösten oder dispergierten Verbindungen auf das Polymere, gegebenenfalls unter nachträglichem Verdunsten des Lösungsmittels, erfolgen. Die neuen Verbindungen der Formel I können auch in Form eines Masterbatches, der diese Verbindungen beispielsweise in einer Konzentration von 2,5 bis 25 Gew.-% enthält, den zu stabilisierenden Kunststoffen zugesetzt werden.

Die erfindungsgemässen Zusammensetzungen können in verschiedenster Form angewendet werden, z. B. als Folien, Fasern, Bändchen, Formmassen, Profile oder als Bindemittel für Lacke, Klebstoffe oder Kitte.

Die folgenden Beispiele erläutern die Erfindung näher :

Beispiel 1

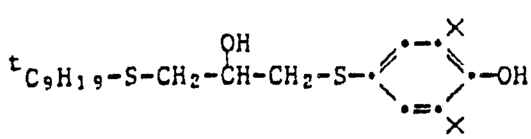

23,8 g (0,1 mol) 4-Mercapto-2,6-di-tert.-butyl-phenol werden in 25 ml Toluol gelöst und nach Zusatz von einer Spatelspitze Natrium-hybrid auf 80 °C erwärmt. Anschliessend werden bei dieser Temperatur 22,7 g (0,105 mol) tert.-Nonyl-glycidyl-thioether unter Rühren zugetropft. Das Reaktionsgemisch wird noch 30 Minuten bei 80 °C nachgerührt, filtriert, und an einem Rotationsdampfer eingeengt.

Ausbeute : 43,1 g = 95 % d.Th., viskose Flüssigkeit

$n_{20}^{D}$ : 1,5369

Beispiele 2-6 : Analog zu Beispiel 1 werden weitere Verbindungen hergestellt, die in Tabelle 1 zusammengestellt sind.

Tabelle 1

| Beispiel Nr. | Formel | Brechungs-index $n_{20}^{D}$ | Schmelz-punkt (°C) |
|---|---|---|---|
| 2 | 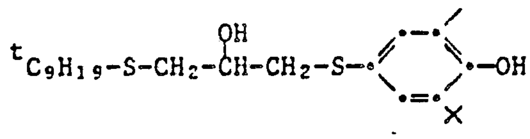 | 1,5458 | |
| 3 | 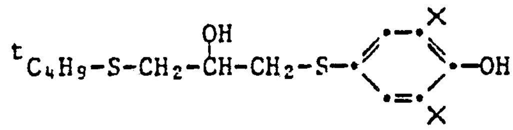 | | 113-115 |
| 4 | 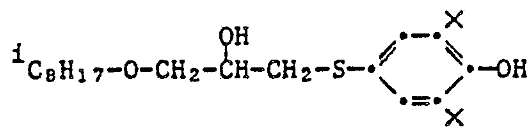 | 1,5166 | |
| 5 | 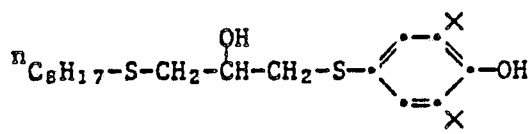 | 1,5327 | |
| 6 | 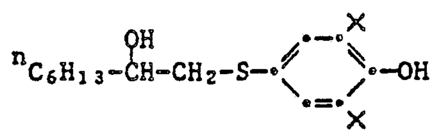 | | 62- 64 |

Beispiel 7

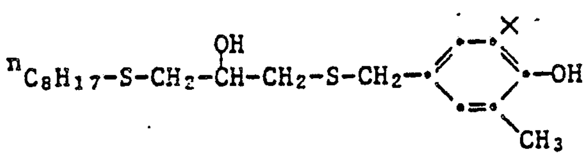

15,8 g (0,075 mol) 4-Mercaptomethyl-2-methyl-6-tert.-butyl-phenol werden in einem 100 ml Zweihalskolben mit einer katalytischen Menge Natriumhydrid versetzt und auf 80 °C erwärmt. Es werden bei dieser Temperatur unter Rühren allmählich 16,2 g (0,08 mol) n-Octyl-glycidylthioether zugegeben. Anschliessend wird das Reaktionsgemisch noch 30 Minuten bei 80 °C nachgerührt und dann aufgearbeitet.

Ausbeute : 32,1 g = 100 % d. Th., viskose Flüssigkeit

$n_{20}^{D}$ : 1,5387

Beispiele 8-9 : Analog zu Beispiel 7 werden weitere Verbindungen hergestellt, die in Tabelle 2 zusammengestellt sind.

(Siehe Tabelle 2 Seite 13 f.)

Tabelle 2

| Beispiel Nr. | Formel | Brechungsindex $n_{20}^{D}$ |
|---|---|---|
| 8 | 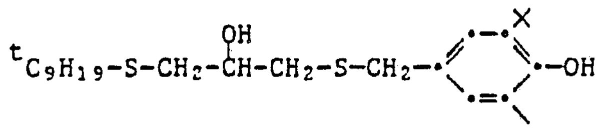 | 1,5418 |
| 9 | 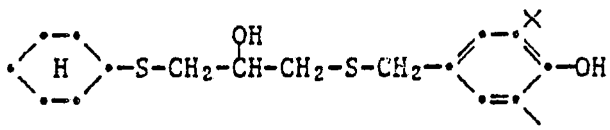 | 1,5666 |

Beispiel 10

19,5 g 2-tert.-Butyl-6-methyl-4-dimethylaminophenol, 23,4 g 1-Mercapto-3-tert.-dodecylthio-2-hydroxypropan und 105 ml Toluol werden in einem mit Thermometer, Stickstoffeinleitung, Rührer und Kühler versehenen 300 ml Dreihalskolben vorgelegt. Die Mischung wird auf 100-105 °C erhitzt und insgesamt 30 Stunden gerührt. Dann wird die Reaktionsmischung zweimal mit je 50 ml 1 N HCl und dreimal mit je 75 ml Wasser gewaschen. Die organische Phase wird über Natriumsulfat getrocknet und nach Entfernung des Trockenmittels wird das Toluol im Vakuum (0,2 mm) bei 60-65 °C abdestilliert. Rückstand : 36,6 g (98 % der Theorie) der Verbindung der Formel

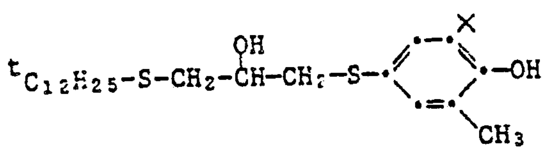

als blassgelber Sirup. Das NMR-Spektrum entspricht der angegebenen Struktur.

Beispiele 11-17 : Analog zu Beispiel 10 werden weitere Verbindungen erhalten, die in Tabelle 3 zusammengestellt sind.

Tabelle 3

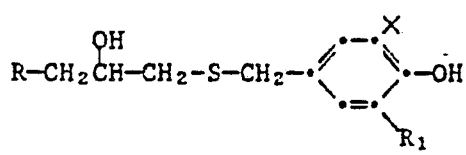

| Beispiel Nr. | $R_1$ | R | Ausbeute (%) | Physikal. Form |
|---|---|---|---|---|
| 11 | $^{t}C_4H_9$ | $^{t}C_{12}H_{25}S-$ | 95 | bernsteinfarbener Sirup |
| 12 | $CH_3$ | $^{t}C_{12}H_{25}S-$ | 98 | gelber Sirup |
| 13 | $^{t}C_4H_9$ | $^{i}C_8H_{17}O-$ | 94 | bernsteinfarbener Sirup |
| 14 | $CH_3$ | $^{i}C_8H_{17}O-$ | 95 | gelber Sirup |

Tabelle 3 (Fortsetzung)

| Beispiel Nr. | $R_1$ | R | Ausbeute (%) | Physikal. Form |
|---|---|---|---|---|
| 15 | $^tC_4H_9$ | $C_8-C_{10}H_{17}-H_{21}C(=O)-O-$ | 89 | bernsteinfarbener Sirup |
| 16 | $CH_3$ | $C_8-C_{10}H_{17}-H_{21}C(=O)-O-$ | 86 | gelber Sirup |
| 17 | $^tC_4H_9$ | $^tC_9H_{19}S-$ | 82 | bernsteinfarbener Sirup |

## Beispiel 18

Mit dem Shell-Vierkugel-Apparat werden nach der ASTM-Standard-Methode D 2783-81 (extreme pressure and wear lubricant test for oils and greases, four ball-machine) folgende Werte bestimmt :

1. W.L. = Weld load (Schweisslast). Das ist die Last, bei der die 4 Kugeln innerhalb von 10 Sekunden zusammenschweissen.
2. W.S.D. = Wear Scar Diameter in mm : Das ist der mittlere Verschleissdurchmesser bei einer Belastung von 400 N während 60 Minuten.

Als Basisöl wird Catenex® P 941 der Firma Shell verwendet. Die Ergebnisse der Tests sind in Tabelle 4 widergegeben.

Tabelle 4

| Additiv Beispiel Nr. | Konzentration [Gew.-%] | WL [N] | WSD [mm] |
|---|---|---|---|
| Basisöl ohne Additiv | - | 1350 | 0,85 |
| 8 | 1,0 | 1600 | 0,55 |
| | 2,5 | 1800 | - |
| 9 | 1,0 | 1600 | 0,60 |
| | 2,5 | 1600 | - |

## Beispiel 19 : Stabilisierung von Polybutadien-Kautschuk (Ofenalterung)

100 g Polybutadien (Diene®35 von Firestone) werden auf einem Mischwalzwerk bei 50 °C während 6 Minuten homogen mit 0,25 % der zu prüfenden Verbindung gemischt. Aus den Walzfellen werden bei 80 °C Platten von 10 mm Dicke gepresst. Eine weitere Platte wird ohne Stabilisator auf dieselbe Weise hergestellt.

Die Stabilitätsprüfung wird durch Hitzealterung in einem Umluftofen bei 80 °C vorgenommen. Als Kriterium dient der während der Ofenalterung auftretende unerwünschte Gelgehalt.

Der Gelgehalt nimmt nach einer Induktionsperiode rasch zu. Als willkürliche Definition der Induktionsperiode dient die Zeit, nach welcher ein Gelgehalt von 15 % erreicht wird. Gemessen wird diese Induktionszeit.

Die Resultate sind in Tabelle 5 zusammengefasst.

Tabelle 5

| Verbindung aus Beispiel Nr. | Induktionszeit bis Gelgehalt von 15 % (in Tagen) |
|---|---|
| ohne | <7 |
| 4 | 17 |
| 13 | 17 |
| 14 | 17 |
| 7 | 25 |
| 6 | 25 |

Beispiel 20 : Stabilisierung von Polybutadien-Kautschuk (Brabender)

100 Teile Polybutadien (Diene®55 von Firestone) werden mit 0,25 % der zu prüfenden Verbindung in einem Brabender-Plastographen bei 160 °C und 60 Umdrehungen pro Minute während 30 Minuten geknetet. Aus dem Verlauf der Drehmomentskurve wird die Induktionszeit ermittelt, d. h. die Zeit in Minuten bis zum Anstieg des Drehmoments nach dem Drehmomentminimum, welcher Anstieg die Vernetzung des Kaustschuks anzeigt.

Die Ergebnisse sind in Tabelle 6 zusammengefasst.

Tabelle 6

| Verbindung aus Beispiel Nr. | Induktionszeit (in Minuten) |
|---|---|
| ohne | 2,0 |
| 1 | 8,0 |
| 2 | 8,8 |

**Patentansprüche**

1. Zusammensetzungen enthaltend ein Schmiermittel oder eine Hydraulikflüssigkeit und mindestens eine Verbindung der Formel I

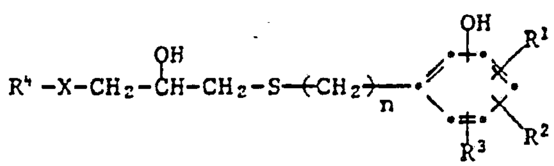

(I)

worin X für —S—, —O—, —CO—O—, —$CH_2$— oder —$NR^5$-steht und $R^5$ Wasserstoff oder $C_1$-$C_8$-Alkyl ist, n die Zahl 0, 1, 2 oder 3 bedeutet, $R^1$ und $R^2$ unabhängig voneinander Wasserstoff oder $C_1$-$C_2$-Alkyl sind, $R^3$ für Wasserstoff oder Methyl steht und $R^4$ $C_1$-$C_{22}$-Alkyl, $C_5$-$C_8$-Cycloalkyl, unsubstituiertes oder mit einem oder zwei $C_1$-$C_8$-Alkylresten substituiertes Aryl, $C_7$-$C_{12}$-Aralkyl oder die Gruppe

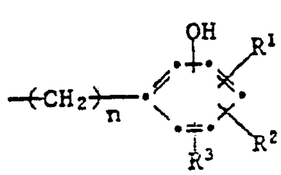

worin $R^1$, R2, $R^3$ und n die angegebene Bedeutung haben, ist.

2. Zusammensetzungen gemäss Anspruch 1, wobei in den Verbindungen der Formel I X —S—, —O—, —$CH_2$— oder —CO—O— bedeutet, n 0 oder 1 ist, $R^1$ und $R^2$ unabhängig voneinander für Wasserstoff, Methyl oder tertiäres $C_4$-$C_8$-Alkyl stehen, $R^3$ Wasserstoff ist und $R^4$ $C_2$-$C_{12}$-Alkyl, $C_5$-$C_6$-Cycloalkyl, unsubstituiertes oder mit einem oder zwei $C_1$-$C_4$-Alkylresten substituiertes Aryl oder $C_7$-$C_{10}$-Aralkyl darstellt.

3. Zusammensetzungen gemäss Anspruch 1, worin X in den Verbindungen der Formel I —S— oder —O— ist.

4. Zusammensetzungen gemäss Anspruch 1, worin $R^4$ $C_1$-$C_{22}$-Alkyl oder $C_5$-$C_8$-Cycloalkyl, vorzugsweise $C_4$-$C_{12}$-Alkyl bedeutet.

5. Zusammensetzungen gemäss Anspruch 1, worin $R^1$ und $R^2$ in den Verbindungen der Formel I unabhängig voneinander Methyl oder tert.-Butyl bedeuten und $R^3$ Wasserstoff ist.

6. Zusammensetzungen gemäss Anspruch 1, worin sich die phenolische Hydroxygruppe in den Verbindungen der Formel I in 4-Stellung befindet und die Reste $R^1$ und $R^2$ in ortho-Stellung zur phenolischen Hydroxygruppe angeordnet sind.

7. Zusammensetzungen nach Anspruch 1, worin X für —S—, —O—, —$CH_2$— oder —CO—O— steht, n 0 oder 1 ist, die phenolische OH-Gruppe in 4-Stellung steht, $R_1$ und $R_2$ die ortho-Stellungen zur phenolischen OH-Gruppe besetzen und unabhängig voneinander $C_1$-$C_6$-Alkyl bedeuten, $R_3$ Wasserstoff ist und $R_4$ $C_2$-$C_{12}$-Alkyl oder Cyclohexyl bedeutet.

8. Zusammensetzungen nach Anspruch 1, enthaltend ein Schmiermittel und mindestens eine Verbindung der Formel I.

9. Verbindungen der Formel I gemäss Anspruch 1, worin X —S—, —O—, —CO—O— oder —$CH_2$— ist, n die Zahl 0, 1, 2 oder 3 bedeutet, $R^1$ und $R^2$ unabhängig voneinander Wasserstoff oder $C_1$-$C_{12}$-Alkyl sind, $R^3$ für Wasserstoff oder Methyl steht und $R^4$ $C_1$-$C_{22}$-Alkyl, $C_5$-$C_8$-Cycloalkyl, unsubstituiertes oder mit einem oder zwei $C_1$-$C_8$-Alkylresten substituiertes Aryl, $C_7$-$C_{12}$-Aralkyl oder, falls n 1, 2 oder 3 ist, auch die Gruppe

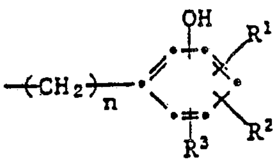

worin $R^1$, $R^2$ und $R^3$ die angegebene Bedeutung haben, ist.

10. Verbindungen gemäss Anspruch 9, worin X —S—, —O—, —$CH_2$— oder —CO—O— bedeutet, n 0 oder 1 ist, $R^1$ und $R^2$ unabhängig voneinander für Wasserstoff, Methyl oder tertiäres $C_4$-$C_8$-Alkyl stehen, $R^3$ Wasserstoff ist und $R^4$ $C_2$-$C_{12}$-Alkyl, $C_5$-$C_6$-Cycloalkyl, unsubstituiertes oder mit einem oder zwei $C_1$-$C_4$-Alkylresten substituiertes Aryl oder $C_7$-$C_{10}$-Aralkyl darstellt.

11. Verbindungen gemäss Anspruch 9, worin $R^4$ $C_1$-$C_{22}$-Alkyl oder $C_5$-$C_8$-Cycloalkyl, vorzugsweise $C_4$-$C_{12}$-Alkyl bedeutet.

12. Verbindungen gemäss Anspruch 9, worin X —O— oder —S— bedeutet.

13. Verbindungen gemäss Anspruch 9, worin die phenolische OH-Gruppe in 4-Stellung steht und $R_1$ und $R_2$ unabhängig voneinander Methyl oder tert.-Butyl bedeuten und die beiden ortho-Stellungen zur OH-Gruppe besetzen.

14. Verbindungen gemäss Anspruch 9, worin X für —S—, —O—, —$CH_2$— oder —CO—O— steht, n 0 oder 1 ist, die phenolische OH-Gruppe in 4-Stellung steht, $R_1$ und $R_2$ die ortho-Stellungen zur phenolischen OH-Gruppe besetzen und unabhängig voneinander $C_1$-$C_6$-Alkyl bedeuten, $R_3$ Wasserstoff ist und $R_4$ $C_2$-$C_{12}$-Alkyl oder Cyclohexyl bedeutet.

15. Verwendung der in Anspruch 9 definierten Verbindungen als Additive für Schmiermittel und Elastomere.

16. Verwendung gemäss Anspruch 15 als Stabilisatoren für Elastomere.

17. Zusammensetzungen, enthaltend ein Schmiermittel oder ein Elastomer und mindestens eine in Anspruch 9 definierte Verbindung.

18. Zusammensetzungen gemäss Anspruch 17, worin das Elastomer ein Polydien, ein halogenhaltiges Polymer oder ein Polyurethan ist.

**Claims**

1. A composition containing a lubricant or a hydraulic fluid and at least one compound of formula I

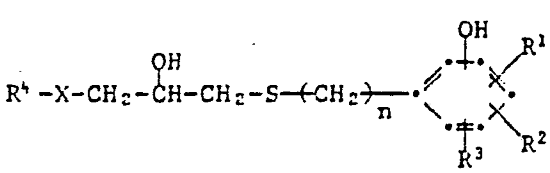

(I)

wherein X is —S—, —O—, —CO—O—, —$CH_2$— or —$NR^5$—, where $R^5$ is hydrogen or $C_1$-$C_8$alkyl, n is the number 0, 1, 2 or 3, $R^1$ and $R^2$ are each independently of the other hydrogen or $C_1$-$C_2$alkyl, $R^3$ is hydrogen or methyl, and $R^4$ is $C_1$-$C_2$alkyl, $C_5$-$C_8$cycloalkyl, aryl which is unsubstituted or substituted by one or two $C_1$-$C_8$alkyl radicals, or is $C_7$-$C_{12}$aralkyl or the group

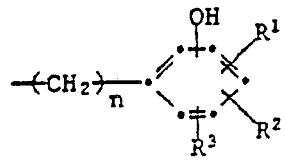

wherein $R^1$, $R^2$, $R^3$ and n have the given meanings.

2. A composition according to claim 1, where in the compounds of formula I, X is —S—, —O—, —$CH_2$— or —CO—O—, n is 0 or 1, $R^1$ and $R^2$ are each independently of the other hydrogen, methyl or tertiary $C_4$-$C_8$alkyl, $R^3$ is hydrogen and $R^4$ is $C_2$-$C_{12}$alkyl, $C_5$-$C_6$cycloalkyl, aryl which is unsubstituted or substituted by one or two $C_1$-$C_4$alkyl radicals, or is $C_7$-$C_{10}$aralkyl.

3. A composition according to claim 1, wherein X in the compounds of formula I is —S— or —O—.

4. A composition according to claim 1, wherein $R^4$ is $C_1$-$C_{22}$alkyl or $C_5$-$C_8$cycloalkyl, preferably $C_4$-$C_{12}$alkyl.

5. A composition according to claim 1, wherein $R^1$ and $R^2$ in the compounds of formula I are each independently of the other methyl or tert-butyl, and $R^3$ is hydrogen.

6. A composition according to claim 1, wherein the phenolic hydroxyl group in the compounds of formula I is in 4-position and the radicals $R^1$ and $R^2$ are located in ortho-position to the phenolic hydroxyl group.

7. A composition according to claim 1, wherein X is —S—, —O—, —$CH_2$ or —CO—O—, n is 0 or 1, the phenolic hydroxyl group is in 4-position, $R^1$ and $R^2$ occupy the positions ortho to the phenolic OH group and are each independently of the other $C_1$-$C_6$alkyl, $R^3$ is hydrogen, and $R^4$ is $C_2$-$C_{12}$alkyl or cyclohexyl.

8. A composition according to claim 1, which contains a lubricant and at least one compound of formula I.

9. A compound of formula I according to claim 1, wherein X is —S—, —O—, —CO—O— or —$CH_2$—, n is the number 0, 1, 2 or 3, $R^1$ and $R^2$ are each independently of the other hydrogen or $C_1$-$C_{12}$alkyl, $C_5$-$C_8$cycloalkyl, aryl which is unsubstituted or substituted by one or two $C_1$-$C_8$alkyl radicals, or is $C_7$-$C_{12}$aralkyl or, if n is 1, 2 or 3, is also the group

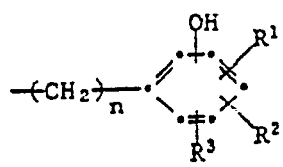

wherein $R^1$, $R^2$ and $R^3$ have the given meanings.

10. A compound according to claim 9, wherein X is —S—, —O—, —$OH_2$— or —OO—O—, n is 0 or 1, $R^1$ and $R^2$ are each independently of the other hydrogen, methyl or tertiary $C_4$-$C_8$alkyl, $R^3$ is hydrogen, and $R^4$ is $C_2$-$C_{12}$alkyl, $C_5$-$C_6$cycloalkyl, aryl which is unsubstituted or substituted by one or two $C_1$-$C_4$alkyl radicals, or is $C_7$-$C_{10}$aralkyl.

11. A compound according to claim 9, wherein $R^4$ is $C_1$-$C_{22}$alkyl or $C_5$-$C_8$cycloalkyl, preferably $C_4$-$C_{12}$alkyl.

12. A compound according to claim 9, wherein X is —O— or —S—.

13. A compound according to claim 9, wherein the phenolic OH group is in 4-position and $R^1$ and $R^2$ are each independently of the other methyl or tert-butyl and occupy both positions ortho to the OH group.

14. A compound according to claim 9, wherein X is —S—, —O—, —$CH_2$— or —CO—O—, n is 0 or 1, the phenolic OH group is in 4-position, $R^1$ and $R^2$ occupy the positions ortho to the phenolic OH group and are each indepentently of the other $C_1$-$C_6$alkyl, $R^3$ is hydrogen, and $R^4$ is $C_2$-$C_{12}$alkyl or cyclohexyl.

15. Use of the compound as defined in claim 9 as an additive for lubricants and elastomers.

16. Use according to claim 15 as stabilizers for elastomers.

17. A composition containing a lubricant or an elastomer and at least one compound as defined in claim 9.

18. A composition according to claim 17, wherein the elastomer is a polydiene, a halogen-containing polymer or a polyurethane.

**Revendications**

1. Compositions qui comprennent un lubrifiant ou un fluide hydraulique avec un ou plusieurs

composés de formule I ci-dessous :

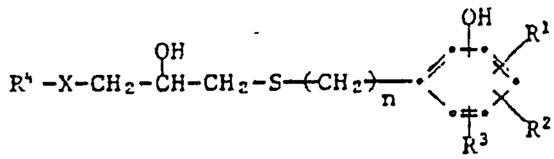

(I)

formule dans laquelle X représente —S—, —O—, —CO—O—, —$CH_2$— ou —$NR^5$, $R^5$ étant l'hydrogène ou un alkyle en $C_1$-$C_8$, n est le nombre 0, 1, 2 ou 3, $R^1$ et $R^2$ désignent chacun, indépendamment l'un de l'autre, l'hydrogène ou un alkyle en $C_1$-$C_{12}$, $R^3$ désigne l'hydrogène ou le groupe méthyle et $R^4$ un alkyle en $C_1$-$C_{22}$, un cycloalkyle en $C_5$-$C_8$, un aryle sans substituants ou avec un ou deux alkyles en $C_1$-$C_8$, un aralkyle en $C_7$-$C_{12}$ ou encore le groupe :

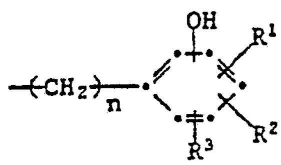

dans lequel les divers symboles ont les significations précédentes.

2. Compositions selon la revendication 1 où, dans les composés de formule I, X représente —S—, —O—, —$CH_2$— ou —CO—O—, n est le nombre 0 ou 1, $R^1$ et $R^2$ désignent chacun, indépendamment l'un de l'autre, l'hydrogène, le groupe méthyle ou un alkyle tertiaire en $C_4$-$C_8$, $R^3$ désigne l'hydrogène et $R^4$ un alkyle en $C_2$-$C_{12}$, un cycloalkyle en $C_5$ ou $C_6$, un aryle pouvant éventuellement porter un ou deux alkyles en $C_1$-$C_4$ ou un aralkyle en $C_7$-$C_{10}$.

3. Compositions selon la revendication 1, X dans les composés de formule I étant le soufre ou l'oxygène.

4. Compositions selon la revendication 1 dans lesquelles $R^4$ est un alkyle en $C_1$-$C_{22}$ ou un cycloalkyle en $C_5$-$C_8$, mais de préférence un alkyle en $C_4$-$C_{12}$.

5. Compositions selon la revendication 1 dans lesquelles $R^1$ et $R^2$ de la formule I sont chacun, indépendamment l'un de l'autre, un méthyle ou le groupe butyle tertiaire, et $R^3$ est l'hydrogène.

6. Compositions selon la revendication 1 dans lesquelles l'hydroxyle phénolique du composé de formule I occupe la position 4 et les radicaux $R^1$ et $R^2$ se trouvent aux positions ortho par rapport à l'hydroxyle phénolique. .

7. Compositions selon la revendication 1 dans lesquelles X représente —S—, —O—, —$CH_2$— ou —CO—O, n est le nombre 0 ou 1, le groupe OH phénolique occupe la position 4, $R^1$ et $R^2$ occupent les positions ortho par rapport au groupe OH phénolique et sont chacun un alkyle en $C_1$-$C_6$, $R^3$ est l'hydrogène et $R^4$ un alkyle en $C_2$-$C_{12}$ ou le groupe cyclohexyle.

8. Compositions selon la revendication 1 qui comprennent un lubrifiant avec un ou plusieurs composés de formule I.

9. Les composés de formule I selon la revendication 1 dans lesquels X est —S—, —O—, —CO—O— ou —$CH_2$—, n le nombre 0, 1, 2 ou 3, $R^1$ et $R^2$ sont chacun, indépendamment l'un de l'autre, l'hydrogène ou un alkyle en $C_1$-$C_{12}$, $R^3$ est l'hydrogène ou le groupe méthyle et $R^4$ un alkyle en $C_1$-$C_{22}$, un cycloalkyle en $C_5$-$C_8$, un aryle pouvant porter éventuellement un ou deux alkyles en $C_1$-$C_8$ ou un aralkyle en $C_7$-$C_{12}$, ou encore, si n = 1, 2 ou 3, un groupe :

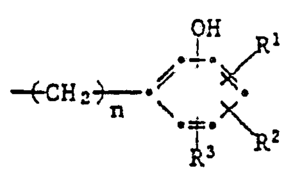

les divers symboles de ce groupe ayant les définitions précédemment données.

10. Composés selon la revendication 9 dans lesquels X est —S—, —O—, —$CH_2$— ou —CO—O—, n le nombre 0 ou 1, $R^1$ et $R^2$ sont chacun l'hydrogène un méthyle ou un alkyle tertiaire en $C_4$-$C_8$, $R^3$ est l'hydrogène et $R^4$ un alkyle en $C_2$-$C_{12}$, un cycloalkyle en $C_5$ ou $C_6$, un aryle pouvant éventuellement porter un ou deux alkyles en $C_1$-$C_4$ ou un aralkyle en $C_7$-$C_{10}$.

11. Composés selon la revendication 9 dans lesquels $R^4$ est un alkyle en $C_1$-$C_{22}$ ou un cycloalkyle en $C_5$-$C_8$, mais de préférence un alkyle en $C_4$-$C_{12}$.

12. Composés selon la revendication 9 dans lesquels X est l'oxygène ou le soufre.

13. Composés selon la revendication 9 dans lesquels le groupe OH phénolique occupe la position 4

et $R^1$ et $R^2$ sont chacun le groupe méthyle ou le groupe butyle tertiaire et occupent les deux positions ortho par rapport au groupe OH.

14. Composés selon la revendication 9 dans lesquels X est —S—, —O—, —$CH_2$— ou —CO—O—, n est le nombre 0 ou 1, le groupe OH phénolique occupe la position 4, $R^1$ et $R^2$ occupent les positions ortho par rapport au groupe OH phénolique et sont chacun un alkyle en $C_1$-$C_6$, $R^3$ est l'hydrogène et $R^4$ un alkyle en $C_2$-$C_{12}$ ou le groupe cyclohexyle.

15. L'emploi des composés définis aux revendications 9 à 14 comme additifs à des lubrifiants ou à des élastomères.

16. Emploi selon la revendication 15 comme stabilisants d'élastomères.

17. Compositions qui comprennent un lubrifiant ou un élastomère avec un ou plusieurs composés tels que définis à la revendication 9.

18. Compositions selon la revendication 17, dans lesquelles l'élastomère est un polydiène, un polymère halogéné ou un polyuréthanne.